# EUROPEAN PATENT APPLICATION

(11) **EP 2 885 977 A1**
(43) Date of publication of application: **24.06.2015**
(21) Application number: 13198686.1
(22) Date of filing: 19.12.2013
(51) Int. Cl.: A23C 9/142, A23L 1/29, A61K 31/702, A61K 35/20

(54) **An oligosaccharide-enriched composition having immune-modulatory and anti-adhesion properties**

(71) Applicant: Agriculture and Food Development Authority (TEAGASC), Co. Carlow (IE); University College Cork, Cork City (IE); UNIVERSITY COLLEGE DUBLIN, Dublin 4 (IE)
(72) Inventor: The designation of the inventor has not yet been filed
(74) Representative: Purdy, Hugh Barry

(57) **Abstract**

This invention provides an oligosaccharide-enriched composition that is derived from milk and that comprises at least 30% oligosaccharides (per 100g of composition dry weight) having a molecular weight range of 300-1200Da, and at least 20% sialyllactose (per 100g of composition dry weight). The oligosaccharide-enriched composition has been found to have a significant immunomodulatory effect (Fig. 1) and has been shown to significantly reduce adhesion of pathogenic bacteria to lung cells and activated pharynx cells, and thereby have a protective effect in humans from respiratory and gastrointestinal infections (Figs 3 and 4). The composition of the invention is derived from a dairy product stream that is substantially free of casein and retains lactose and oligosaccharides, for example demineralised whey powder, demineralised whey, whey permeate, or mother liquor. The dairy product stream is subjected to a micro filtration step to remove whey protein and bacteria, the permeate is then subjected to an ultrafiltration step to enrich for oligosaccharides, and the permeate from the ultrafiltration step is enriched in oligosaccharides having a molecular weight of300-1200Da, using a chromatographic step.

## Description

### Background to the Invention

Cows milk protein allergy (CMPA) affects 2.2 % to 7.5 % of infants worldwide and is a growing public health problem in Western Europe and USA. In Europe, 20 to 30 % of infants have been diagnosed with an atopic (allergic) disease. The majority of the first atopic responses are directed towards food proteins that are observed during the first months of life with a rate of resolution at 56% after 1 year and 87% resolved after 3 years determined from milk allergy birth cohort study. Consequently, the most common allergy is the one directed against cow's milk proteins for infants below 3 years. All existing hypoallergenic formula solutions adopt "avoidance" strategies based on the extensive hydrolysis of whey or casein proteins (using proteolytic bacteria or enzymes), or use of amino acid formula which prevent the IgE mediated response associated with allergy. They are therefore positioned as allergy prevention formulae. For example, Danone launched a hypoallergenic weaning food "Neocate Nutra" as a medical food for infants with severe CMPA in 2009. None of the currently available solutions adopt a "treatment" strategy, whereby a functional formula is specifically developed to induce oral tolerance over time by re-addressing the immune imbalance of infants diagnosed with allergy.

Respiratory tract infections (RTIs) are associated with almost 50% of all paediatric consultations in industrialized countries. Moreover, in developed countries, such as the US, 6% of infants younger than 1 year are hospitalised with lower respiratory tract diseases on an annual basis (Bachrach et al., (2003) Archives of Pediatrics and Adolescent Medicine, 157, 237-243). Acute otitis media (etiological agent *Streptococcus pneumoniae*) is one of the most frequent childhood diseases and prevention of infection is generally difficult. Indeed, epidemiological studies on otitis media have reported that 62% of infants will have at least 1 episode of acute otitis media during the first year of life, while 17% will have at least 3 episodes (Duncan et al. (1993) Pediatrics, 91, 867-872; Teele et al.(1989) Journal of Infectious Diseases, 160, 83-94). A recent review (Duijts et al. (2009) Maternal and Child Nutrition, 5, 199-210) reported that breastfeeding protects infants against RTIs. Recently, oligosaccharides in human milk have been implicated in this protective role. Human milk oligosaccharides display structural homology to host cell receptors and thus function as receptor decoys which pathogens can bind to instead of the host (Newburg (2009) Journal of Animal Science, 87, 26-34). Several *in vitro* studies have documented this ability of milk oligosaccharides to interfere with these specific host pathogen interactions (Coppa et al. (2006) Pediatric Research, 59, 377-382; Cravioto et al. (1991) Journal of Infectious Diseases, 163, 1247-1255; Simon et al. (1997) Infection and Immunity, 65 750-757). Moreover, it is well documented that adhesion of respiratory, oral and enteric bacteria is required for colonization of the body and subsequent development of disease. Therefore, prevention of adhesion at an early stage following exposure of the host to pathogens should prevent disease. Currently there is no infant formula commercially available which incorporates milk oligosaccharides, to aid in the protection of infants from respiratory infections.

It is an object of the invention to overcome at least one of the above-referenced problems.

### Statements of Invention

This invention provides an oligosaccharide-enriched composition that is derived from milk and that comprises of at least 23 % or 30% oligosaccharides (at least 23g or 30g per 100g of composition dry weight) having a molecular weight range of 300-1200Da, and at least 20% sialyllactose (at least 20g per 100g of composition dry weight). The oligosaccharide-enriched composition has been found to have a significant immunomodulatory effect (Fig. 2) and has been shown to significantly reduce adhesion of pathogenic bacteria to activated pharynx cells and lung cells, and thereby have a protective effect in humans from respiratory and gastrointestinal infections (Fig. 3a,b, Fig. 4).

The composition of the invention is derived from a commercial milk by-product that is substantially free of casein and retains lactose and oligosaccharides, for example demineralised whey powder, whey permeate, or mother liquor. The dairy product stream is subjected to a microfiltration step to remove whey protein and bacteria, the permeate is then subjected to an ultrafiltration step to enrich for oligosaccharides, and the permeate from the ultrafiltration step is enriched in oligosaccharides having a molecular weight of 300-1200Da. One method of enrichment involves fractionation of the permeate according to size, and pooling of fractions with low peptide (i.e.< 1 g/L) and lactose concentration (i.e.< 100 mg/L). In the methods described below, size exclusion chromatography is employed for the latter step, although other methods may be employed for the separation. The resultant composition is enriched in sialyllactose (3'-sialyllactose and 6'-sialyllactose), which is the predominant oligosaccharide in bovine milk, is substantially free of lactose and protein, and was additionally found to contain a number of additional oligosaccharides including both sialylated and neutral oligosaccharides.

In a first aspect, the invention provides a milk derived oligosaccharide enriched composition comprising at least 30% oligosaccharides (dry weight) having a molecular weight range of 300-1200 Da, at least 20% sialyllactose (dry weight), and wherein the composition is substantially free of lactose and protein.

The term "at 20% sialyllactose (dry weight)" as applied herein means that the composition comprises at least 20g sialyllactose per 100g of dry composition.

In this specification, the term "oligosaccharide-enriched composition" should be understood to mean a composition which when in a dry form comprises at least 30%, 35%, 40% or 45% oligosaccharides (dry weight).

Preferably, substantially all of the oligosaccharides in the composition, for example at least 90%, 92%, 95% or 97% of the total detectable oligosaccharide, are oligosaccharides ranging in size from 300-1200 Da as determined by the method outlined by Marino et al. (2011) Glycobiology 21; 1317-1330.

The term "milk derived" should be understood to mean that the composition is obtained from commercial milk sources or by-products, typically obtained from a whey fraction, for example whey permeate, mother liquor, demineralised whey or demineralised whey powder. The term "milk" should be primarily understood to mean bovine milk, but may also include milk from other mammals such as sheep or goats.

The term "substantially free" as applied to the lactose content of the composition should be understood to mean less than 2% (dry weight), and preferably less than 1% (dry weight) of the composition is lactose. The term "substantially free" as applied to protein means that less than 1% (dry weight), and preferably less that 0.1% (dry weight) of the composition comprises protein having a molecular weight of greater than 1kDa. Ideally, the composition is free of protein having a molecular weight of greater than 1kDa.

Typically, the composition comprises a peptide content of less than 15% (dry weight), and ideally less than 10% (dry weight), wherein at least 90% of the peptides have a molecular weight of less than or equal to 1kDa. Suitably, the composition comprises a peptide content of about 9% (dry weight).

Suitably, the composition comprises a sialyllactose content of at least 20% w/w and above as determined by HPLC using the technique described below.

Preferably, the composition is enriched in oligosaccharides selected from 3'-sialyllactose (Table 1, peak 13), 6'-sialyllactose (Table 1, peak 19), and and there should be at least 6 other oligosaccharide structures present (Table 1, peak 9).

The composition of the invention typically has a significant immunomodulatory effect and/or significant anti-adhesion effect. The term "significant immunomodulatory effect" should be understood to mean that the composition is capable of up-regulating secretion of the antiinflammatory and immunomodulatory cytokine IL-10 by APCs (BMDCs and the murine macrophages J774 cell line) and down-regulates IL-4 production by Th2 cells and preventing chemical degranulation by mast cells. The term "significant anti-adhesion effect" should be understood to mean that the composition is capable of significantly reducing adhesion of pathogenic bacteria to lung cells and activated pharynx cells as described below.

The composition is preferably in dry form, for example a particulate product such as a powder, flakes, pellets and the like. The dry composition may be generated by means of any suitable drying technique, such as spray drying, drum drying or freeze-drying. The invention also relates to liquid compositions comprising the composition of the invention suspended or solubilised in a suitable liquid such as water.

The invention also provides a food product comprising milk derived oligosaccharide enriched composition of the invention. Typically, the food product is selected from a dairy product, beverage, infant food, cereal, biscuits, confectionary, cakes, food supplements, dietary supplements, animal feeds, poultry feeds. Preferably the food product is an infant formula. Typically, the food product comprises from 0.1-10.0%, 0.1-5.0%, 0.1-1.0% (w/w) of the oligosaccharide-enriched composition of the invention.

The invention also relates to an infant formula comprising a milk derived oligosaccharide enriched composition according to the invention, typically in an amount of 0.1 to 1.0% (w/v), ideally about 0.3-0.5% (w/v) of reconstituted formula.

The invention also relates to a milk derived oligosaccharide enriched composition, or a food product of the invention, or an infant formula of the invention, for use in a method of: (1) treating or preventing inflammation or an inflammatory disease in a mammal; (2) treating or preventing a respiratory or gastrointestinal infection in a mammal; (3) treating or prevening allergy, especially dairy product induced allergy; or (4) inducing oral tolerance to allergy, especially dairy product induced allergy, especially cows milk protein allergy (CMPA).

Typically, the mammal is a higher mammal, especially a human.

In this specification, the term "inflammatory disease" should be understood to mean a disease that exhibits inflammation as part of the pathology of the disease, including for example allergies, autoimmune diseases, rheumatoid arthritis, inflammatory bowel disease, atherosclerosis, multiple sclerosis, cardiovascular disease, diabetes and hypersensitivity disorders of the immune system, for example allergies, for example cows milk protein allergy.

In this specification, the term "respiratory or gastrointestinal infection" refers to infections of the respiratory tract or gastrointestinal tract, especially infections caused by *Streptococcus pneumoniae, Pseudomonas aeruginosa, Haemophilus influenzae, Moraxella catharralis* and Respiratory Syncytial Virus

The invention also relates to a method of generating a milk derived oligosaccharide enriched composition comprising at least 20% sialyllactose, and that is substantially free of lactose and protein, the method comprising the steps of:
- providing a dairy product stream that is substantially free of casein and retains lactose and oligosaccharides;
- treating the dairy product stream to one or more membrane filtration step to provide an intermediate oligosaccharide enriched composition;
- separating the intermediate oligosaccharide enriched composition into fractions having different saccharide content;
- combining the fractions that are low in lactose and peptides to provide the milk derived oligosaccharide enriched composition; and
- optionally, drying the composition.

In the specification, the term "low in lactose" means less than 100mg/L lactose, and the term "low in peptides" means less than 1 g/L peptides.

In this specification, the term "dairy product stream" should be understood to mean a dairy product stream obtained from commercial milk which retains lactose and oligosaccharides and from which caseins have been removed. Examples include whey permeate, mother liquor, demineralised whey and liquid streams obtained using demineralised whey powder..

Suitably, the step of treating the dairy product stream to one or more membrane filtration step to provide the intermediate oligosaccharide enriched composition comprises the steps of:
- treating the dairy product stream to an initial microfiltration step top provide a permeate and a retentate; and
- treating the permeate to a ultrafiltration step to provide a permeate and a retentate in which the retentate is the intermediate oligosaccharide enriched composition.

Suitably, the microfiltration stem employs a membrane having 0.1mm molecular weight cut-off.

Preferably, the dairy product stream is selected from demineralised whey, whey permeate, or mother liquor. Suitably, the dairy product stream has a dry matter content of 1-30% (1-20g/100ml).

### Brief Description of the Figures

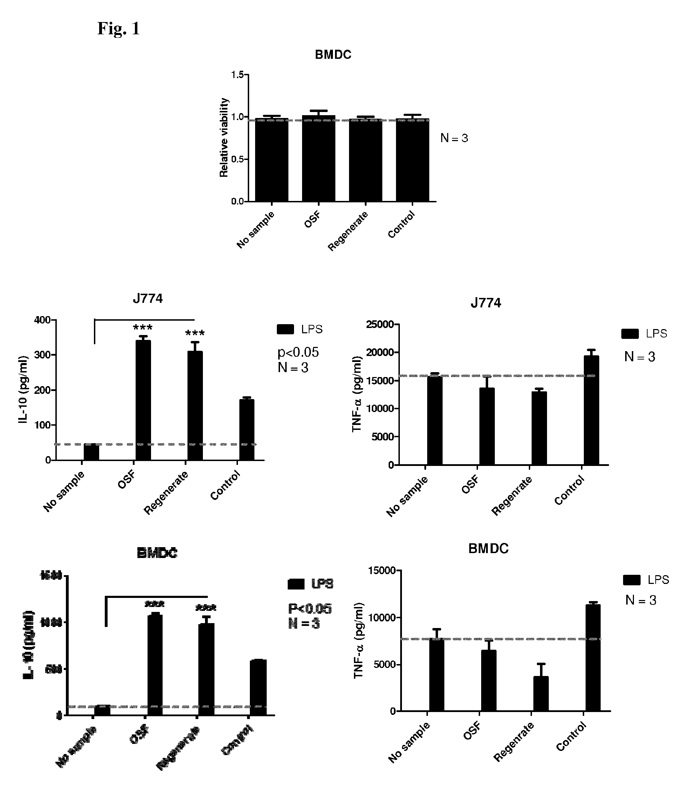

Fig. 1 OSF does not affect the relative viability of BMDCs, up-regulates secretion of the antiinflammatory and immunomodulatory cytokine IL-10 by APCs (BMDCs and the murine macrophages J774 cell line) and down-regulates the pro-inflammatory production of TNF-α in both APC models.

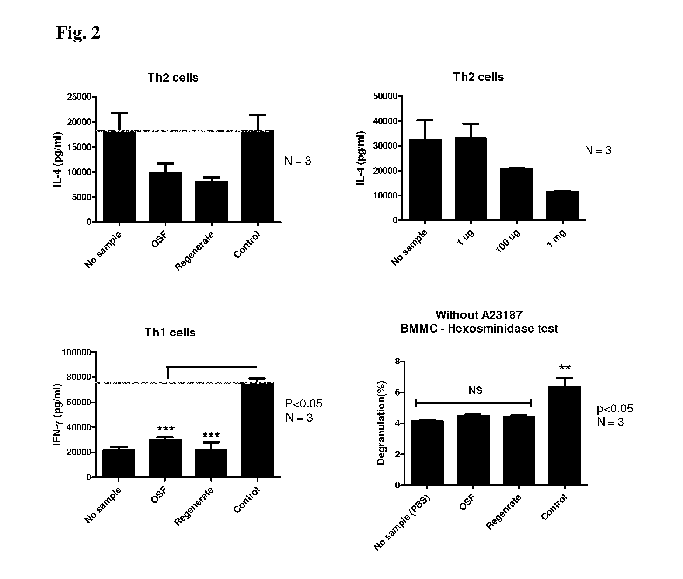

Fig. 2 OSF modulates IL-4 production by Th2 cells in a dose-dependant manner, inhibits IFN-γ release from Th1 cells and prevents chemical degranulation by mast cells. Therefore, OSF significantly interferes with the mechanistic of hypersensitivity to cow's milk proteins.

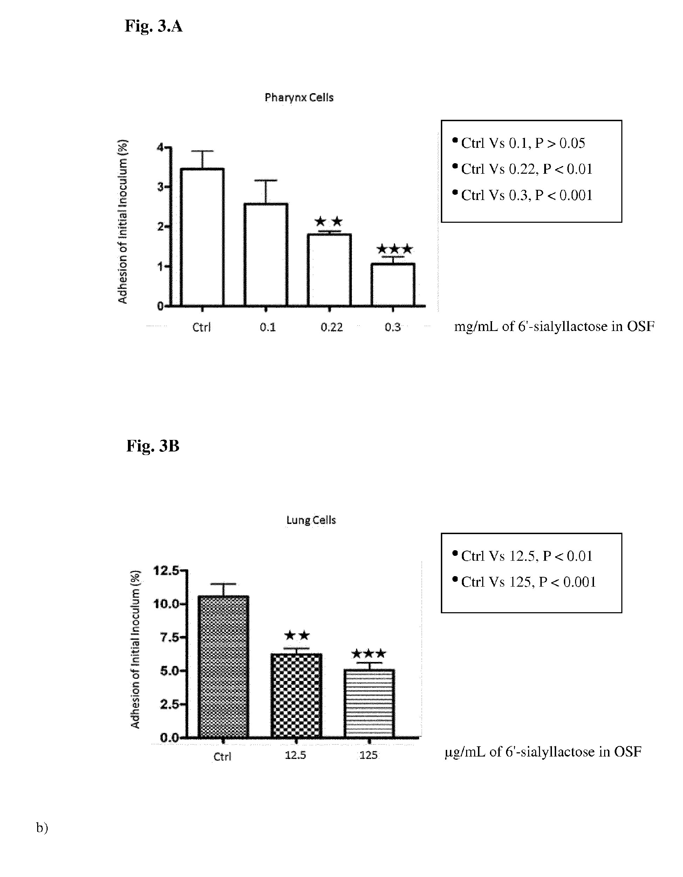

Fig. 3 The effect of the concentration of OSF on adhesion of *S*. *pneumoniae* to activated pharynx cells (a) and lung cells (b)..In (a) the maximum concentration of OSF was chosen in order to match the minimum concentration of 6SL found in human breast milk, 0.3 mg/mL. In (b) the maximum concentration of OSF was chosen in order to match the maximum estimated concentration of 6SL in infant blood, 125 µg/mL. The control was *S*. *pneumoniae* in the absence of OSF (Ctrl)

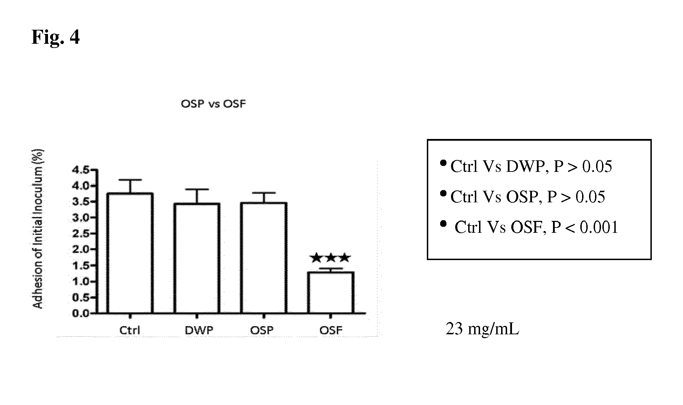

Fig. 4 The effect of demineralised whey powder (DWP), intermediate oligosaccharide enriched composition (OSP) and oligosaccharide enriched composition (OSF) on adhesion of *S. pneumoniae* to activated pharynx cells. The concentration (23 mg/mL) for each sample was chosen in order that the concentration of 6SL in the OSF matched the minimum concentration of 6SL found in human breast milk, 0.3 mg/mL. The control was *S*. *pneumoniae* in the absence of any source of oligosaccharides (Ctrl)

### Detailed Description of the Invention

### Materials and Methods

Milk oligosaccharides were enriched from demineralised whey powder, which was resuspended in water to give a final volume of 2428 L at 5% solids. The starting material was treated through consecutive microfiltration, ultrafiltration and diafiltration steps, as described by WO 2007/051475 A1. The steps are described in more detail below.

### Clarification demineralised whey by microfiltration

Demineralised whey powder was re-suspended in water to have a final volume of 2428 L at 5% solid. The demineralised whey solution was microfiltered through 0.1 mm molecular weight cut-off (MWCO) membranes using a pilot scale Tetra Alcross MSF19 microfiltration plant (Tetra Pak Filtration Systems A/S, Aarhus, Denmark). The plant has 2 loops, each equipped with tubular ceramic multichannel membranes (19 filter channels, 102 cm long, and 3 mm channel diameter) enclosed in acid-proof stainless housings. The total membrane surface area of this plant was 12.5 m². Membranes were cleaned according to the manufacturer's instructions before and after use. Filtration was performed at 20°C at the following pressures: retentate inlet, 29.3 kPa; retentate outlet, 11.7 kPa; permeate inlet, 24.1 kPa, and permeate outlet, 15.8 kPa. This step removed the residual insoluble material and bacteria from the demineralised whey, leaving soluble mineral salts, lactose and oligosaccharides in the microfiltrate.

### Fractionation and enrichment of milk oligosaccharides by membrane Filtration

The microfiltrate from the microfiltration step was utilized as the feed to the ultrafiltration plant for fractionation and enrichment of milk oligosaccharides. The process was carried out using six, 1-kDa MWCO, spirally wound membranes (Desal Membrane Products from GE Osmonics, Minnetonka, MN, USA) with a total membrane surface area of 30 m². Ultrafiltration was carried out in batch mode at about 10°C, with inlet and outlet pressures of 551.6 kPa and 413.6 kPa, respectively. The purpose of this step was to separate and concentrate oligosaccharides from lactose and mineral salts. The membrane allowed retention of oligosaccharides and permeation of lactose and minerals into the filtrate. The oligosaccharides-rich retentate was then diafiltered with reverse osmosis water in five consecutive batch diafiltration steps to further wash out the lactose into the permeate, thus allowing further enrichment of the retained oligosaccharides. The final diafiltered retentate was concentrated by evaporation at 50°C and spray dried with inlet and outlet temperatures of 120°C and 80°C, respectively. This process yielded 2.5 kg of bovine milk oligosaccharides-rich powder (OSP). Lactose and sialyllactose, being the smallest oligosaccharides present in milk and whey streams for which commercial standard exist, were chosen as markers of oligosaccharide retention and enrichment during processing.

### Quantification of lactose and sialyllactose by HPLC

Lactose was quantified by HPLC using an HPX-87C Carbohydrate column (300 x 7.8 mm) (Aminex, Bio-Rad, UK) and a refractive index detector. The elution was obtained in isocratic conditions using 9 mM sulphuric acid for 30 min. Both sialyllactose isomers (3'-sialyllactose and 6'-sialyllactose) were quantified by High pH Anion Exchange Chromatography with Pulsed Amperometric Detection, using a CarboPac PA 100 (250 x 4 mm) connected to a CarboPac PA 100 guard column and equipped with an electrochemical detector (Dionex Corporation, Sunnyvale, CA). Elution was carried out with the following gradient: 100 mM NaOH (Eluent A) and 100 mM NaOH, 500 mM NaAc (Eluent B) (t = 0-3 min 95% Eluent A; t = 3-13 min 88% Eluent A; t = 13-30 min 50% Eluent A; t = 30-45 min equilibrated at 95% Eluent A).

### Enrichment of oligosaccharides using size exclusion chromatography

OSP was purified as follows:-A BioGel P2 size exclusion column, 5cm x 92 cm with a volume of 1805 mL was used to produce OSF. 50 mL of a 20% solution of OSP was injected on to the BioGelP2 column and eluted with water at a flow rate of 3 mL/min. The fractions (14 mL) were analysed for lactose, OS and peptides concentration. The fractions with low peptides and lactose concentrations were pooled, freeze dried and analysed for sialyllactose, lactose and peptide concentration. The composition of the resulting oligosaccharides enriched composition of the invention (OSF) was assessed for the presence of 3'- and 6'-sialyllactose, lactose, peptides, and ash (minerals), which were respectively 24g/100g sialyllactose (as sum of 3'-sialylactose and 6'-sialyllactose), 0.9 g/100g, 9 g/100g and 13 g/100g.
The structural characterisation of the oligosaccharides in OSF was performed by NIBRT according to Marino et al. (2011) Glycobiology 21; 1317-1330. The oligosaccharide profile of the OSF produced according to this embodiment is provided in Table 1 below and comprises:
- 19 different identified oligosaccharides in the 300-1200 Da range;
- 47.8% 3'-sialyllactose (as a % of total detectable oligosaccharide);
- 7.4% 6'-sialyllactose;
- 23.8% GalNAc(α1-3)Gal(β1-4)Glc α3-*N*-acetylgalactosaminylactose;
- Unidentified neutral di- or tri- saccharides;
- Unidentified acidic oligosaccharides.

### Adhesion Assays

### Organisms and growth conditions

The *Streptococcus pneumoniae* strain ATCC BAA-255 (*S. pneumoniae R6*) was obtained from the American Type Culture Collection. *S. pneumoniae R6* was stored in Todd Hewitt broth (Becton Dickinson and Company, France) containing 10% (v/v) glycerol at -80°C and cultured directly from storage into the same broth (0.2% inoculum) at 37°C with 5% CO₂ until an optical density (600nm) of 0.8 was reached.

### Culture of Detroit 562 and A549 cell lines

Adherent Detroit 562 (pharynx) and A549 (lung) cells were purchased from the American Type Culture Collection. These cell lines were chosen because of their routine use in previous publications. The Detroit 562 cells were cultured in tissue culture flasks in presence of Eagle's Minimum Essential Medium (EMEM) supplemented with 10% (v/v) of fetal bovine serum (FBS) and 1% of Penicillin-Streptomycin. The A549 cells were cultured in tissue culture flasks in presence of Dulbecco's Modified Eagle's Medium (DMEM) supplemented with 10% (v/v) of fetal bovine serum (FBS) and 1% of Penicillin-Streptomycin. Cells were incubated at 37°C in an atmosphere of 5% CO₂ and subcultured 1:6 every 3-4 days. For the purpose of the adhesion assay, the cells were washed twice with PBS before incubation with 0.25% trypsin/EDTA solution. Trypsination was stopped by adding 8-10 mL of fresh medium with the exclusion of antibiotics. The cells were then seeded in 12-well plates to a density of 5x10⁵/well. After overnight incubation as described above, the spent medium was replaced with 1 mL of DMEM or EMEM supplemented with 2% FBS. After a further overnight incubation as described above, the cells were used in the adhesion assay.

### Adhesion assays

*S. pneumoniae R6* was grown to an optical density (OD₆₀₀) between 0.6 -0.8 and adjusted to an OD of 0.23 by centrifugation (4500g, 8min), washed twice in phosphate buffered saline (PBS; Sigma, Steinheim, Germany) and resuspended in EMEM or DMEM tissue culture media (2% FBS; further referred to as non-supplemented media) supplemented with OSF at concentrations ranging from 12.5 µg/mL to 0.3 mg/mL of 6'-siallylactose found within OSF. Non-supplemented media was used as a control. The bacteria were exposed to the oligosaccharides at room temperature for 30 min.
Four hours prior to addition of bacteria to eukaryotic cells, eukaryotic cells were incubated with the cytokine interleukin 1β, in order to activate the cells with the obj ective of mimicking a typical infection response that might occur *in vivo.* Directly prior to exposure of eukaryotic cells to *S. pneumoniae R6,* eukaryotic cells were washed three times with appropriate tissue culture media to remove interleukin 1β. Bacterial exposure to eukaryotic cells was for 30 mins at 37°C with 5% CO₂. After 30 min, the wells were washed five times with PBS to remove any non-adherent bacteria and lysed with 1mL of PBS containing 0.025% Triton X100 (Sigma, Steinheim, Germany) for 30 min at 37°C on a shaking platform at 100 revolutions per minute to ensure maximal recovery of viable bacterial cells. The lysates were serially diluted and enumerated by spread-plating on sheep blood agar plates and incubated overnight at 37°C with 5% CO₂. Aliquots of the experimental inocula were retained, diluted and plated to determine original CFU/mL. The results were expressed as adherent bacteria as a percentage of the original inoculum, thereby accounting for variations in the original inocula between groups. Percentage adherent = [CFU/mL of recovered adherent bacteria ÷ CFU/mL of inoculum] X 100. Each adhesion assay was conducted independently in triplicate over 3 successive passages of Detroit 562 and A549 cells.

### Immunomodulation Assays

### Mice

Balb/C and C57BL/6 mice aged between 6-10 weeks supplied by Charles River Biotech (U.K.) were kept under standard conditions in the DCU Biological Resource Unit according to the regulations of the European Union and the Irish Department of Health.

### Splenocyte purification

Female Balb/C mice were sacrificed by cervical dislocation and spleens removed and put in cold RPMI-1640 (Life Technologies). In the laminar flow, spleens were gently disrupted on a Falcon® 40 µm sterile cell strainer (Coming Life Sciences) and washed through with RPMI-1640 then centrifuged at 1200 rpm for 5 mins. Cells were resuspended in 10 mL of medium, passed through another Falcon® 40 µm filter and counted before spinning at 1200 rpm for 5 mins.

### CD4 + T-cell isolation

Medium was discarded and cells were prepared in PBS, 2% FCS, 5% rat serum at a concentration of 1 x 10⁸/mL. CD4+ T cells were negatively isolated using the protocol provided by EasySep Mouse CD4+ T Cell Enrichement Kit (Stemcell Technologies). After purification, cells were washed with medium then resuspended in filtered RPMI-1640 supplemented with 10% heat inactivated foetal calf serum (Life Technologies), 50 µM β-mercaptoethanol and 5000 units/mL penicillin/5000 µg/mL streptomycin at a concentration of 2 x 10⁶ cells/mL.

### T-cell polarisation

Ninety-six-well flat-bottomed plates were coated with 50 µL of 5 µg/mL anti-mouse CD3 (BD Biosciences) in sterile PBS overnight at 4° C. The next day, wells were washed with sterile medium and left to dry. Then naive TC were cultured at 2 x 10⁶ cells/mL under the following conditions: Th1, 10 ng/mL of recombinant mouse IL-12, 20 ng/mL of recombinant mouse IL-2, 10 µg/mL of anti-IL-4 and 2.5 µg/mL of anti-mouse CD28 (BD Biosciences); Th2, 10 ng/mL of recombinant mouse IL-4, 10 µg/mL of anti-IFN-γ and 2.5 µg/mL of anti-mouse CD28. Both Th1 and Th2 were differentiated in the presence or absence of 1 mg/mL of OSF. After three days of polarised stimulation, cells were resuspended in fresh complete medium and transferred onto a fresh anti-CD3-coated plate in a final culture volume of 200 µL with 2.5 µg/mL of anti-mouse CD28 in the presence or absence of 1mg/mL of the OSF. Cell supernatants were collected 72 h after secondary stimulation and stored at -20° C for ELISA analysis.

### BMDCs isolation and differentiation

Bone marrow-derived immature dendritic (BMDCs) cells were generated from the femoral and tibia bone marrow cells of Balb/C mice and differentiated in complete RPMI-1640 supplemented with 10% heat inactivated foetal calf serum, 2% penicillin/streptomycin solution (v/v) and 10% supernatant from a GM-CSF-expressing cell line (J558-GM-CSF) for 3 days. Then supernatants were carefully removed and replaced with fresh medium containing 10% GM-CSF cell supernatant. Cell number and viability were assessed using trypan blue staining. On day 7 of culture, BMDCs were collected, counted and plated on twenty-four well plates at a concentration of 1 x 10⁶ cell/mL in a final culture volume of 500 µL. Cells were pre-incubated with 1 mg/mL OSF for 1 hour then challenged with 100 ng/mL of LPS as an inflammatory stimulus. After 24 hrs, supernatants were collected and stored at -20° C for cytokine quantification using ELISA.

### BMMCs isolation and saturation

Bone marrow-derived mast cells (BMMCs) were generated from the femoral and tibia bone marrow cells of C57BL/6 mice and maintained in complete IMDM in the presence of 10% heat-inactivated FCS, 100 u/mL penicillin/streptomycin and 30% WEHI-3 conditioned IMDM-medium (ATTC TIB-68) as a source of the murine growth-factor IL-3 for 4 weeks (Vukman et al. (2013). J. Immunol. Mar; 15; 190 (6):2873-9). Cell number and viability were monitored using trypan blue staining.

### Measurement of β-Hexosaminidase release from BMMCs

β-hexosaminidase release from mast cells was measured as previously described (Vukman et al. (2012). Inflamm Res. Jan; 61(1):79-85. BMMCs were treated with OSF. After 24 hours mast cells were washed twice with PBS, resuspended in Tyrodes buffer (130 mM NaCl, 10 mM HEPES, 1 mM MgCl2, 5 mM KCl, 1.4 mM CaC12, 5.6 mM glucose and 1 mg/mL BSA, pH7.4) and seeded in a 96-well round-bottom plate at a density of 4 x 10⁵cells/200 µL followed by stimulation with A23187 (1 µM) or PBS in the presence or absence of OSF for 1 hour at 37° C. Cell pellets were solubilised in Tyrodes buffer supplemented with 1% Triton-X, and cell lysates and supernatants were incubated separately with 4 mm p-nitrophenyl-N-acetyl-b-d-glucopyranoside in 0.2 M disodium phosphate and 0.4 M citrate buffer pH4.5 for 1 h at 37° C. The production of β-hexosaminidase-mediated p-nitrophenol was terminated by adding 150 µL 0.2 M glycine (pH 10.7) and quantified by absorbance at 405 nm. The percentage of β-hexosaminidase release indicating the extent of degranulation was calculated as the ratio of p-nitrophenol absorbance in the supernatants and the sum of absorbance in the supernatant and cell lysates.

### J774 cell line

The murine macrophage cell line J774.1 was purchased from the ECACC and referred to solely as J774 throughout. Cells were maintained in complete RPMI-1640 supplemented with 10% FCS and 1% penicillin/streptomycin in 75 cm2 flasks. Cells monolayers were passaged at a confluency of 80% every 3 to 4 days. Cells were detached using sterile scrapers, washed, resuspended at a concentration of 1 x 10⁶ cell/mL and seeded on twenty-four well plates in a final culture volume of 500 µL. Cells were pre-incubated with 1 mg/mL OSF for 1 hour then stimulated with 100 ng/mL of LPS. After 24 h, supernatants were collected and stored at -20° C for cytokine measurements using ELISA.

### MTS assay and ELISA

Relative viability of cells was assessed using the CellTiter 96® AQueous One Solution Cell Proliferation Assay (MTS) from Promega in accordance to manufacturer's guidelines. DuoSet® ELISA Development Systems were purchased from R&D Systems and performed in compliance with the manufacturer's recommended protocol.

The invention is not limited to the embodiments hereinbefore described which may be varied in construction and detail without departing from the spirit of the invention.

**Table 1. Structural assignment of oligosaccharides in oligosaccharide enriched composition of the invention (OSF)**

| Peak Nr | m/z observed | m/z theoretical | UXOF Symbol structural assignment | GU value | Relative % UPLC-HILIC FLD(*) |
|---|---|---|---|---|---|
| 1 | - | - | monosaccharides | 1.00 | - |
| 2 | | | | 1.03 | - |
| 3 | 502.22 | 502.20 | 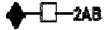 | 1.88 | 0.7 |
| 4 | 461.21 | 461.18 | 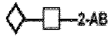 | 1.95 | 2.5 |
| 5 | - | - | neutral di- or tri-saccharides | 2.28 | - |
| 6 | - | - | acidic di- or tri-saccharides | 2.33 | 0.1 |
| 7 | | | | 2.36 | 0.02 |
| 8 | 607.27 | 607.24 | 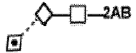 | 2.49 | 0.7 |
| | 794.32 | 794.28 | 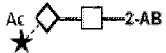 | | |
| 9 | 664.29 | 664.26 | 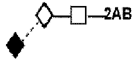 | 2.71 | 23.8 |
| 10 | 664.29 | 664.26 | 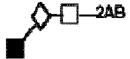 | 2.83 | 1.3 |
| 11 | 623.26 | 623.23 | 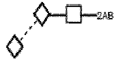 | 2.89 | 1.3 |
| 12 | 623.26 | 623.23 | 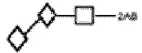 | 2.98 | 9.6 |
| | | | major component: | | |
| 13 | 752.31 | 752.27 | 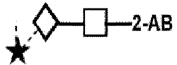 | 3.15 | 47.8 |
| | 623.26 | 623.23 | minor component: | | |
| | | | 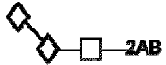 | | |
| 14 | - | - | acidic tri- or tetrasaccharides | 3.30 | 0.2 |
| 15 | | | | 3.34 | 0.1 |
| 16 | | | | 3.38 | 0.1 |
| 17 | 793.35 | 793.30 | 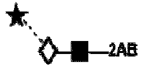 | 3.48 | 0.7 |
| 18 | 768.30 | 768.27 | 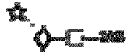 | 3.50 | 0.4 |
| 19 | 752.31 | 752.27 | 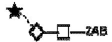 | 3.56 | 7.4 |
| 20 | 826.36 | 826.31 | 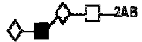 | 3.63 | 0.6 |
| 21 | 1085.47 | 1085.38 | 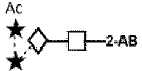 | 3.84 | 0.1 |
| 22 | 955.39 | 955.35 | 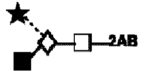 | 3.91 | 0.3 |
| 23 | 914.37 | 914.33 | 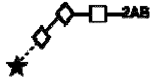 | 4.01 | 1.3 |
| 24 | - | - | acidic oligosaccharide | 4.11 | 0.03 |
| 25 | 914.37 | 914.33 | 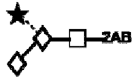 | 4.33 | 0.2 |
| 26 | 1043.44 | 1043.37 | 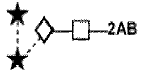 | 4.58 | 0.4 |
| 27 | 1029.45 | 1029.39 | 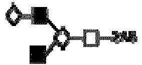 | 4.69 | - |
| 28 | 988.43 | 988.36 | 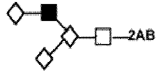 | 4.84 | 0.1 |
| 29 | - | - | acidic oligosaccharide | 5.47 | 0.2 |

| | | | | | |
|---|---|---|---|---|---|
| (*) relative proportion represents area of peaks compared to total peak area in UPLC-HILIC-FLD chromatograms. | | | | | |

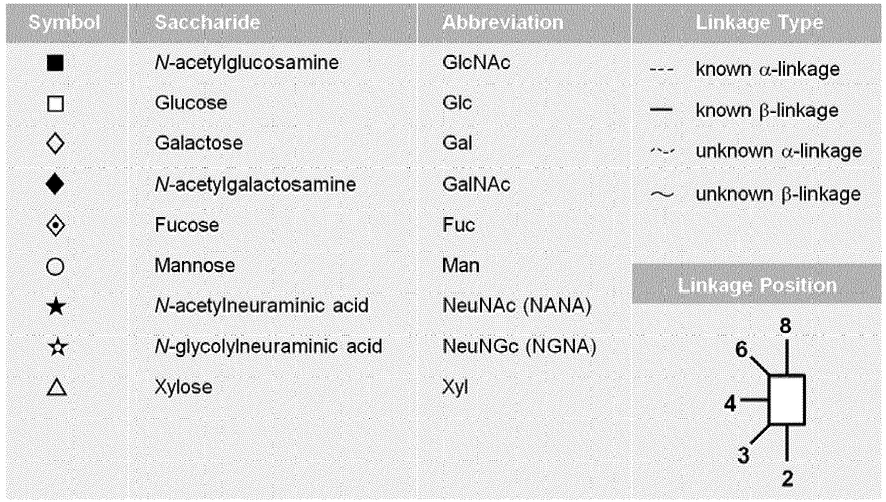

## Claims

1. A milk derived oligosaccharide-enriched composition comprising at least 30% oligosaccharides (dry weight) having a size range of 300-1200 Da, at least 20% sialyllactose (dry weight), and which is substantially free of lactose and protein.

2. A composition according to Claim 1 having less than 1% lactose and free of protein, and in which the milk is bovine milk.

3. A composition according to Claim 1 or 2 in which the oligosaccharide-enriched composition is derived from milk.

4. A composition according to any preceding Claim having a peptide content of less than 15% (dry weight), in which at least 90% of the peptides have a molecular weight of less than 1000Da.

5. A composition according to any preceding Claim and comprising at least 35% oligosaccharides (dry weight) having a size range of 300-1200 Daltons.

6. A composition according to any preceding Claim having a significant immunomodulatory effect and significant anti-adhesion effect.

7. A composition according to any preceding Claim and obtained by a method of:
providing a dairy product stream that is substantially free of casein and retains lactose and oligosaccharides;
treating the dairy product stream to one or more membrane filtration step to provide an intermediate oligosaccharide enriched composition;
separating the intermediate oligosaccharide enriched composition into fractions having different saccharide content;
combining the fractions that are low in lactose and peptides to provide the milk derived oligosaccharide enriched composition; and
optionally, drying the composition..

8. A food product comprising a milk derived oligosaccharide enriched composition according to any of Claims 1 to 6 or obtained by a method of Claim 7.

9. A food product as claimed in Claim 8 in which the food product is an infant formula.

10. A milk derived oligosaccharide enriched composition according to any of Claims 1 to 6, for use in a method of treating or preventing inflammation or an inflammatory disease in a human.

11. A milk derived oligosaccharide enriched composition according to any of Claims 1 to 6, for use in a method of treating or preventing a respiratory or gastrointestinal infection in a human.

12. A composition suitable for being sprayed onto the pharynx comprising a bovine-milk derived oligosaccharide enriched composition according to any of Claims 1 to 6 dispersed in a suitable liquid vehicle.

13. A milk derived oligosaccharide enriched composition according to any of Claims 1 to 6, for use in a method of inducing oral tolerance to non-human milk protein allergy in a human.

14. A method of generating a milk derived oligosaccharide enriched composition comprising at least 20% sialyllactose, and that is substantially free of lactose and protein, the method comprising the steps of:
■ providing a dairy product stream that is substantially free of casein and retains lactose and oligosaccharides;
■ treating the dairy product stream to one or more membrane filtration step to provide an intermediate oligosaccharide enriched composition;
■ separating the intermediate oligosaccharide enriched composition into fractions having different saccharide content;
■ combining the fractions that are low in lactose and peptides to provide the milk derived oligosaccharide enriched composition; and
■ optionally, drying the composition.

15. A method according to Claim 14 in which the step of treating the dairy product stream to one or more membrane filtration step to provide the intermediate oligosaccharide enriched composition comprises the steps of:
■ treating the dairy product stream to an initial microfiltration step to provide a permeate and a retentate; and
■ treating the permeate to a ultrafiltration step to provide a permeate and a retentate in which the retentate is the intermediate oligosaccharide enriched composition.
